# EUROPEAN PATENT SPECIFICATION

(11) **EP 1 390 504 B1**
(45) Date of publication and mention of the grant of the patent: **13.04.2011**
(21) Application number: 02740582.8
(22) Date of filing: 10.05.2002
(51) Int. Cl.: C12N 15/31, C07K 14/34, C12P 13/12, C12R 1/15, C12Q 1/68, A23K 1/16

(54) **NUCLEOTIDE SEQUENCES WHICH CODE FOR THE METD GENE**
METD KODIERENDE NUKLEOTIDSEQUENZEN
SEQUENCES NUCLEOTIDIQUES CODANT POUR LE GENE METD

(30) Priority: 30.05.2001 DE 10126164
(43) Date of publication of application: 25.02.2004
(73) Proprietor: Evonik Degussa GmbH, 45128 Essen (DE)
(72) Inventor: REY, Daniel, 33818 Leopoldshöhe (DE); RÜCKERT, Christian, 33335 Gütersloh (DE); KALINOWSKI, Jörn, 33615 Bielefeld (DE); PÜHLER, Alfred, 33739 Bielefeld (DE); BATHE, Brigitte, 33154 Salzkotten (DE); HUTHMACHER, Klaus, 63571 Gelnhausen (DE); PFEFFERLE, Walter, 33790 Halle (Westf.) (DE)
(86) International application number: PCT/EP2002/005152
(87) International publication number: WO 2002/097096

(56) References cited:
- EP-A- 1 108 790
- US-A- 3 729 381
- TAUCH A ET AL: "The erythromycin resistance gene of the Corynebacterium xerosis R-plasmid pTP10 also carrying chloraphenicol, kanamycin and tetracycline resistances is capable of transposition in Corynebacterium glutamicum" PLASMID, vol. 33, - 1995 pages 168-179, XP001135120
- SU Y ET AL: "CONSTRUCTION OF LYSINE-PRODUCING STRAINS BY GENE DISRUPTION AND REPLACEMENT IN BREVIBACTERIUM DIVARICATUM" PROCEEDINGS OF THE NATIONAL SCIENCE COUNCIL. REPUBLIC OF CHINA. PART B. LIFE SCIENCES, NATIONAL SCIENCE COUNCIL, TAIPEI, TW, vol. 19, no. 2, April 1995 (1995-04), pages 113-122, XP001061837 ISSN: 0255-6596

## Description

### Field of the Invention

The invention provides nucleotide sequences from coryneform bacteria which code for the metD gene and a process for the preparation of amino acids using bacteria in which the metD gene is attenuated.

### Prior Art

L-Amino acids, in particular L-methionine, are used in human medicine and in the pharmaceuticals industry, in the foodstuffs industry and very particularly in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites or are auxotrophic for metabolites of regulatory importance and which produce amino acids are obtained in this manner.

Methods of the recombinant DNA technique have also been employed for some years for improving the strain of Corynebacterium strains which produce L-amino acid, by amplifying individual amino acid biosynthesis genes and investigating the effect on the amino acid production.

### Object of the Invention

The object of the invention is to provide new measures for improved fermentative preparation of amino acids.

### Description of the invention

Where L-amino acids or amino acids are mentioned in the following, this means one or more amino acids, including their salts, chosen from the group consisting of L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine. L-Methionine is particularly preferred.

When L-methionine or methionine are mentioned in the following, the salts, such as e.g. methionine hydrochloride or methionine sulfate are also meant by this.

The invention provides an isolated polynucleotide from coryneform bacteria, comprising a polynucleotide sequence which codes for the metD gene, chosen from the group consisting of
a) polynucleotide which is identical to the extent of at least 70% to a polynucleotide which codes for a polypeptide which comprises the amino acid sequence of SEQ ID No. 2,
b) polynucleotide which codes for a polypeptide which comprises an amino acid sequence which is identical to the extent of at least 70% to the amino acid sequence of SEQ ID No. 2,
c) polynucleotide which is complementary to the polynucleotides of a) or b),
d) polynucleotide comprising at least 15 successive nucleotides of the polynucleotide sequence of a), b) or c),
the polypeptide preferably having the activity of the transcription regulator MetD.

Transcriptional regulators are proteins which are able to increase or decrease the transcription level of specific genes by binding at certain DNA regions. It has been found, that the regulators have a specific structure called helix-turn-helix-motif. The invention provides the function of the transcriptional regulator metD as the repression of genes which are involved in L-amino acid biosyntheses in particular the biosynthesis of L-methionine. The attenuation of the transcriptional regulator metD improves the production of L-methionine in coryneform bacteria.

The invention also provides the above-mentioned polynucleotide, this preferably being a DNA which is capable of replication, comprising:
(i) the nucleotide sequence, shown in SEQ ID No.1, or
(ii) at least one sequence which corresponds to sequence (i) within the degeneracy of the genetic code, or
(iii) at least one sequence which hybridizes with the sequences complementary to sequences (i) or (ii), and optionally
(iv) sense mutations of neutral function in (i) which do not modify the activity of the protein/ polypeptide.

Finally, the invention also provides polynucleotides chosen from the group consisting of
a) polynucleotides comprising at least 15 successive nucleotides chosen from the nucleotide sequence of SEQ ID No. 1 between positions 1 and 313,
b) polynucleotides comprising at least 15 successive nucleotides chosen from the nucleotide sequence of SEQ ID No. 1 between positions 314 and 1024,
c) polynucleotides comprising at least 15 successive nucleotides chosen from the nucleotide sequence of SEQ ID No. 1 between positions 1025 and 1322.

The invention also provides:
a polynucleotide, in particular DNA, which is capable of replication and comprises the nucleotide sequence as shown in SEQ ID No.1;
a polynucleotide which codes for a polypeptide which comprises the amino acid sequence as shown in SEQ ID No. 2;
a vector containing parts of the polynucleotide according to the invention, but at least 15 successive nucleotides of the sequence claimed,
and coryneform bacteria in which the metD gene is attenuated, in particular by an insertion or deletion.

The invention also provides polynucleotides, which substantially comprise a polynucleotide sequence, which are obtainable by screening by means of hybridization of a corresponding gene library of a coryneform bacterium, which comprises the complete gene or parts thereof, with a probe which comprises the sequence of the polynucleotide according to the invention according to SEQ ID No.1 or a fragment thereof, and isolation of the polynucleotide sequence mentioned.

Polynucleotides which comprise the sequences according to the invention are suitable as hybridization probes for RNA, cDNA and DNA, in order to isolate, in the full length, nucleic acids or polynucleotides or genes which code for the transcription regulator MetD or to isolate those nucleic acids or polynucleotides or genes which have a high similarity with the sequence of the metD gene. They are also suitable for incorporation into so-called "arrays", "micro arrays" or "DNA chips" in order to detect and determine the corresponding polynucleotides.

Polynucleotides which comprise the sequences according to the invention are furthermore suitable as primers with the aid of which DNA of genes which code for the transcription regulator MetD can be prepared by the polymerase chain reaction (PCR).

Such oligonucleotides which serve as probes or primers comprise at least 25, 26, 27, 28, 29 or 30, preferably at least 20, 21, 22, 23 or 24, very particularly preferably at least 15, 16, 17, 18 or 19 successive nucleotides. Oligonucleotides with a length of at least 31, 32, 33, 34, 35, 36, 37, 38, 39 or 40 or at least 41, 42, 43, 44, 45, 46, 47, 48, 49 or 50 nucleotides are also suitable. Oligonucleotides with a length of at least 100, 150, 200, 250 or 300 nucleotides are optionally also suitable.

"Isolated" means separated out of its natural environment.

"Polynucleotide" in general relates to polyribonucleotides and polydeoxyribonucleotides, it being possible for these to be non-modified RNA or DNA or modified RNA or DNA.

The polynucleotides according to the invention include a polynucleotide according to SEQ ID No. 1 or a fragment prepared therefrom and also those which are at least 70% to 80%, preferably at least 81% to 85%, particularly preferably at least 86% to 90% and very particularly preferably at least 91%, 93%, 95%, 97% or 99% identical to the polynucleotide according to SEQ ID No. 1 or a fragment prepared therefrom.

"Polypeptides" are understood as meaning peptides or proteins which comprise two or more amino acids bonded via peptide bonds.

The polypeptides according to the invention include a polypeptide according to SEQ ID No. 2, in particular those with the biological activity of the transcription regulator MetD, and also those which are at least 70% to 80%, preferably at least 81% to 85%, particularly preferably at least 86% to 90% and very particularly preferably at least 91%, 93%, 95%, 97% or 99% identical to the polypeptide according to SEQ ID No. 2 and have the activity mentioned.

The invention furthermore relates to a process for the preparation of amino acids chosen from the group consisting of L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine using coryneform bacteria which in particular already produce amino acids and in which the nucleotide sequences which code for the metD gene are attenuated, in particular eliminated or expressed at a low level.

The term "attenuation" in this connection describes the reduction or elimination of the intracellular activity of one or more enzymes (proteins) in a microorganism which are coded by the corresponding DNA, for example by using a weak promoter or using a gene or allele which codes for a corresponding enzyme or protein with a low activity or inactivates the corresponding gene or enzyme (protein), and optionally combining these measures.

By attenuation measures, the activity or concentration of the corresponding protein is in general reduced to 0 to 75%,0 to 50%, 0 to 25%, 0 to 10% or 0 to 5% of the activity or concentration of the wild-type protein or the activity of the starting microorganism.

The increase of the protein concentration can be analyzed by 1- and 2-dimensional protein gel electrophoresis followed by optical identification of the protein concentration while using specific computer software. A common method to prepare protein gels using coryneform bacteria and to identify the proteins is described by Hermann et al. (Electrophoresis, 22:1712-23 (2001)).

The concentration of the protein can be also analyzed by Western blot hybridization techniques while using specific antibodies (Sambrook et al., Molecular Cloning: A Laboratory Manual. 2nd Ed., Cold Spring Harbor Laboratory Press, Cold Spring Harbor, N.Y., 1989) and subsequent optical evaluation with computer software commonly used for analyzing of protein concentrations (Lohaus und Meyer (1998) Biospektrum 5:32-39; Lottspeich (1999) Angewandte Chemie 111:2630-2647). The activity of DNA binding proteins can be measured by DNA band shift assays (also known as gel retardation (Wilson et al. (2001) Journal of Bacteriology 183:2151-2155). The influence of DNA binding proteins on gene expression can be identified by well described reporter gene assays (Sambrook et al., Molecular cloning: a laboratory manual. 2nd Ed. Cold Spring Harbor Laboratory Press, Cold Spring Harbor, N.Y., 1989).

The microorganisms provided by the present invention can prepare amino acids from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular of the species Corynebacterium glutamicum (C. glutamicum), are in particular the known wild-type strains
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 and
Brevibacterium divaricatum ATCC14020
or L-amino acid-producing mutants or strains prepared therefrom, as, for example the methionine-producing strain
Corynebacterium glutamicum ATCC21608.

The new metD gene from C. glutamicum which codes for the transcription regulator MetD has been isolated.

To isolate the metD gene or also other genes of C. glutamicum, a gene library of this microorganism is first set up in Escherichia coli (E. coli). The setting up of gene libraries is described in generally known textbooks and handbooks. The textbook by Winnacker: Gene und Klone, Eine Einführung in die Gentechnologie [Genes and Clones, An Introduction to Genetic Engineering] (Verlag Chemie, Weinheim, Germany, 1990), or the handbook by Sambrook et al.: Molecular Cloning, A Laboratory Manual (Cold Spring Harbor Laboratory Press, 1989) may be mentioned as an example. A well-known gene library is that of the E. coli K-12 strain W3110 set up in λ vectors by Kohara et al. (Cell 50, 495 -508 (1987)). Bathe et al. (Molecular and General Genetics, 252:255-265, 1996) describe a gene library of C. glutamicum ATCC13032, which was set up with the aid of the cosmid vector SuperCos I (Wahl et al., 1987, Proceedings of the National Academy of Sciences USA, 84:2160-2164) in the E. coli K-12 strain NM554 (Raleigh et al., 1988, Nucleic Acids Research 16:1563-1575).

Börmann et al. (Molecular Microbiology 6(3), 317-326 (1992)) in turn describe a gene library of C. glutamicum ATCC13032 using the cosmid pHC79 (Hohn and Collins, 1980, Gene 11, 291-298).

To prepare a gene library of C. glutamicum in E. coli it is also possible to use plasmids such as pBR322 (Bolivar, 1979, Life Sciences, 25, 807-818) or pUC9 (Vieira et al., 1982, Gene, 19:259-268). Suitable hosts are, in particular, those E. coli strains which are restriction- and recombination-defective, such as, for example, the strain DH5αmcr, which has been described by Grant et al. (Proceedings of the National Academy of Sciences USA, 87 (1990) 4645-4649). The long DNA fragments cloned with the aid of cosmids or other λ vectors can then in turn be subcloned and subsequently sequenced in the usual vectors which are suitable for DNA sequencing, such as is described e. g. by Sanger et al. (Proceedings of the National Academy of Sciences of the United States of America, 74:5463-5467, 1977).

The resulting DNA sequences can then be investigated with known algorithms or sequence analysis programs, such as e.g. that of Staden (Nucleic Acids Research 14, 217-232(1986)), that of Marck (Nucleic Acids Research 16, 1829-1836 (1988)) or the GCG program of Butler (Methods of Biochemical Analysis 39, 74-97 (1998)).

The new DNA sequence of C. glutamicum which codes for the metD gene and which, as SEQ ID No. 1, is a constituent of the present invention has been found. The amino acid sequence of the corresponding protein has furthermore been derived from the present DNA sequence by the methods described above. The resulting amino acid sequence of the metD gene product is shown in SEQ ID No. 2. It is known that enzymes endogenous in the host can split off the N-terminal amino acid methionine or formylmethionine of the protein formed.

Coding DNA sequences which result from SEQ ID No. 1 by the degeneracy of the genetic code are also a constituent of the invention. In the same way, DNA sequences which hybridize with SEQ ID No. 1 or parts of SEQ ID No. 1 are a constituent of the invention. Conservative amino acid exchanges, such as e.g. exchange of glycine for alanine or of aspartic acid for glutamic acid in proteins, are furthermore known among experts as "sense mutations" which do not lead to a fundamental change in the activity of the protein, i.e. are of neutral function. Such mutations are also called, inter alia, neutral substitutions. It is furthermore known that changes on the N and/or C terminus of a protein cannot substantially impair or can even stabilize the function thereof. Information in this context can be found by the expert, inter alia, in Ben-Bassat et al. (Journal of Bacteriology 169:751-757 (1987)), in O'Regan et al. (Gene 77:237-251 (1989)), in Sahin-Toth et al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2 are also a constituent of the invention.

In the same way, DNA sequences which hybridize with SEQ ID No. 1 or parts of SEQ ID No. 1 are a constituent of the invention. Finally, DNA sequences which are prepared by the polymerase chain reaction (PCR) using primers which result from SEQ ID No. 1 are a constituent of the invention. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology 41: 255-260 (1991)). The hybridization takes place under stringent conditions, that is to say only hybrids in which the probe and target sequence, i. e. the polynucleotides treated with the probe, are at least 70% identical are formed. It is known that the stringency of the hybridization, including the washing steps, is influenced or determined by varying the buffer composition, the temperature and the salt concentration. The hybridization reaction is preferably carried out under a relatively low stringency compared with the washing steps (Hybaid Hybridisation Guide, Hybaid Limited, Teddington, UK, 1996).

A 5x SSC buffer at a temperature of approx. 50°C - 68°C, for example, can be employed for the hybridization reaction. Probes can also hybridize here with polynucleotides which are less than 70% identical to the sequence of the probe. Such hybrids are less stable and are removed by washing under stringent conditions. This can be achieved, for example, by lowering the salt concentration to 2x SSC and optionally subsequently 0.5x SSC (The DIG System User's Guide for Filter Hybridisation, Boehringer Mannheim, Mannheim, Germany, 1995) a temperature of approx. 50°C - 68°C being established. It is optionally possible to lower the salt concentration to 0.1x SSC. Polynucleotide fragments which are, for example, at least 70% or at least 80% or at least 90% to 95% or at least 96% to 99% identical to the sequence of the probe employed can be isolated by increasing the hybridization temperature stepwise from 50°C to 68°C in steps of approx. 1 - 2°C. It is also possible to isolate polynucleotide fragments which are completely identical to the sequence of the probe employed. Further instructions on hybridization are obtainable on the market in the form of so-called kits (e.g. DIG Easy Hyb from Roche Diagnostics GmbH, Mannheim, Germany, Catalogue No. 1603558).

Instructions for amplification of DNA sequences with the aid of the polymerase chain reaction (PCR) can be found by the expert, inter alia, in the handbook by Gait: Oligonucleotide Synthesis: A Practical Approach (IRL Press, Oxford, UK, 1984) and in Newton and Graham: PCR (Spektrum Akademischer Verlag, Heidelberg, Germany, 1994).

It has been found that coryneform bacteria produce amino acids in an improved manner after attenuation of the metD gene.

To achieve an attenuation, either the expression of the metD gene or the regulatory properties of the enzyme protein can be reduced or eliminated. The two measures can optionally be combined.

The reduction in gene expression can take place by suitable culturing or by genetic modification (mutation) of the signal structures of gene expression. Signal structures of gene expression are, for example, repressor genes, activator genes, operators, promoters, attenuators, ribosome binding sites, the start codon and terminators. The expert can find information on this e.g. in the patent application WO 96/15246, in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in Pátek et al. (Microbiology 142: 1297 (1996)), Vasicova et al. (Journal of Bacteriology 181: 6188 (1999)) and in known textbooks of genetics and molecular biology, such as e.g. the textbook by Knippers ("Molekulare Genetik [Molecular Genetics]", 6th edition, Georg Thieme Verlag, Stuttgart, Germany, 1995) or that by Winnacker ("Gene und Klone [Genes and Clones]", VCH Verlagsgesellschaft, Weinheim, Germany, 1990).

Mutations which lead to a change or reduction in the catalytic properties of enzyme proteins are known from the prior art; examples which may be mentioned are the works by Qiu and Goodman (Journal of Biological Chemistry 272: 8611-8617 (1997)), Sugimoto et al. (Bioscience Biotechnology and Biochemistry 61: 1760-1762 (1997)) and Möckel ("Die Threonindehydratase aus Corynebacterium glutamicum: Aufhebung der allosterischen Regulation und Struktur des Enzyms [Threonine dehydratase from Corynebacterium glutamicum: Canceling the allosteric regulation and structure of the enzyme]", Reports from the Jülich Research Center, Jül-2906, ISSN09442952, Jülich, Germany, 1994). Summarizing descriptions can be found in known textbooks of genetics and molecular biology, such as e.g. that by Hagemann ("Allgemeine Genetik [General Genetics]", Gustav Fischer Verlag, Stuttgart, 1986).

Possible mutations are transitions, transversions, insertions and deletions. Depending on the effect of the amino acid exchange on the enzyme activity, "missense mutations" or "nonsense mutations" are referred to. Insertions or deletions of at least one base pair (bp) in a gene lead to frame shift mutations, as a consequence of which incorrect amino acids are incorporated or translation is interrupted prematurely. Deletions of several codons typically lead to a complete loss of the enzyme activity. Instructions on generation of such mutations are prior art and can be found in known textbooks of genetics and molecular biology, such as e.g. the textbook by Knippers ("Molekulare Genetik [Molecular Genetics]", 6th edition, Georg Thieme Verlag, Stuttgart, Germany, 1995), that by Winnacker ("Gene und Klone [Genes and Clones]", VCH Verlagsgesellschaft, Weinheim, Germany, 1990) or that by Hagemann ("Allgemeine Genetik [General Genetics]", Gustav Fischer Verlag, Stuttgart, 1986).

A common method of mutating genes of C. glutamicum is the method of "gene disruption" and "gene replacement" described by Schwarzer and Pühler (Bio/Technology 9, 84-87 (1991)).

In the method of gene disruption a central part of the coding region of the gene of interest is cloned in a plasmid vector which can replicate in a host (typically E. coli), but not in C. glutamicum. Possible vectors are, for example, pSUP301 (Simon et al., Bio/Technology 1, 784-791 (1983)), pK18mob or pK19mob (Schäfer et al., Gene 145, 69-73 (1994)), pK18mobsacB or pK19mobsacB (Jäger et al., Journal of Bacteriology 174: 5462-65 (1992)), pGEM-T (Promega Corporation, Madison, WI, USA), pCR2.1-TOPO (Shuman (1994). Journal of Biological Chemistry 269:32678-84; US Patent 5,487,993), pCR®Blunt (Invitrogen, Groningen, Holland; Bernard et al., Journal of Molecular Biology, 234: 534-541 (1993)) or pEM1 (Schrumpf et al, 1991, Journal of Bacteriology 173:4510-4516). The plasmid vector which contains the central part of the coding region of the gene is then transferred into the desired strain of C. glutamicum by conjugation or transformation. The method of conjugation is described, for example, by Schafer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross-over" event, the coding region of the gene in question is interrupted by the vector sequence and two incomplete alleles are obtained, one lacking the 3_{'} end and one lacking the 5' end. This method has been used, for example, by Fitzpatrick et al. (Applied Microbiology and Biotechnology 42, 575-580 (1994)) to eliminate the recA gene of C. glutamicum.

In the method of "gene replacement", a mutation, such as e.g. a deletion, insertion or base exchange, is established in vitro in the gene of interest. The allele prepared is in turn cloned in a vector which is not replicative for C. glutamicum and this is then transferred into the desired host of C. glutamicum by transformation or conjugation. After homologous recombination by means of a first "cross-over" event which effects integration and a suitable second "cross-over" event which effects excision in the target gene or in the target sequence, the incorporation of the mutation or of the allele is achieved. This method was used, for example, by Peters-Wendisch et al. (Microbiology 144, 915 - 927 (1998)) to eliminate the pyc gene of C. glutamicum by a deletion.

A deletion, insertion or a base exchange can be incorporated into the metD gene in this manner.

In addition, it may be advantageous for the production of L-amino acids to enhance, in particular over-express, one or more enzymes of the particular biosynthesis pathway, of glycolysis, of anaplerosis, of the citric acid cycle, of the pentose phosphate cycle, of amino acid export and optionally regulatory proteins, in addition to the attenuation of the metD gene.

The term "enhancement" in this connection describes the increase in the intracellular activity of one or more enzymes (proteins) in a microorganism which are coded by the corresponding DNA, for example by increasing the number of copies of the gene or of the genes or alleles, using a potent promoter or using a gene or allele which codes for a corresponding enzyme (protein) having a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on the activity or concentration of the wild-type protein or the activity of the starting microorganism.

The use of endogenous genes is in general preferred. The term "endogenous genes" or "endogenous nucleotide sequences" is understood to mean the genes or nucleotide sequences present in the population of a species.

Thus, for the preparation of L-amino acids, in addition to the attenuation of the metD gene at the same time one or more of the genes chosen from the group consisting of
- the gap gene which codes for glyceraldehyde 3-phosphate dehydrogenase (Eikmanns (1992), Journal of Bacteriology 174:6076-6086),
- the tpi gene which codes for triose phosphate isomerase (Eikmanns (1992), Journal of Bacteriology 174:6076-6086),
- the pgk gene which codes for 3-phosphoglycerate kinase (Eikmanns (1992), Journal of Bacteriology 174:6076-6086),
- the zwf gene which codes for glucose 6-phosphate dehydrogenase (JP-A-09224661, WO 01/70995),
- the pyc gene which codes for pyruvate carboxylase (EP-A-1083225),
- the lysC gene which codes for a feed-back resistant aspartate kinase (Accession No.P26512; EP-B-0387527; EP-A-0699759; WO 00/63388),
- the hom gene which codes for homoserine dehydrogenase (EP-A 0131171),
- the metA gene which codes for homoserine O-acetyltransferase (ACCESSION Number AF052652),
- the metB gene which codes for cystathionine gamma-synthase (ACCESSION Number AF126953),
- the aecD gene which codes for cystathionine gamma-lyase (ACCESSION Number M89931),
- the metY gene which codes for 0-acetylhomoserine sulfhydrylase (DE 10043334, DSM 13556),
- the glyA gene which codes for serine hydroxymethyltransferase (JP-A-08107788)
can be enhanced, in particular over-expressed.

It may furthermore be advantageous for the production of amino acids, in addition to the attenuation of the metD gene, at the same time for one or more of the genes chosen from the group consisting of
- the pck gene which codes for phosphoenol pyruvate carboxykinase (EP-A-1094111),
- the pgi gene which codes for glucose 6-phosphate isomerase (EP-A-1087015, WO 01/07626),
- the poxB gene which codes for pyruvate oxidase (EP-A-1096013),
- the thrB gene which codes for homoserine kinase (ACCESSION Number P08210),
- the thrC gene which codes for threonine synthase (ACCESSION Number P23669),
- the metK gene which codes for methionine adenosyltransferase (ACCESSION Number AJ290443)
- the ddh gene which codes for meso-diaminopimelate D-dehydrogenase (ACCESSION Number Y00151),
to be attenuated, in particular for the expression thereof to be reduced.

In addition to the attenuation of the metD gene it may furthermore be advantageous for the production of amino acids to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The invention also provides the microorganisms prepared according to the invention, and these can be cultured continuously or discontinuously in the batch process (batch culture) or in the fed batch (feed process) or repeated fed batch process (repetitive feed process) for the purpose of production of L-amino acids. A summary of known culture methods is described in the textbook by Chmiel (Bioprozesstechnik 1. Einfuhrung in die Bioverfahrenstechnik [Bioprocess Technology 1. Introduction to Bioprocess Technology] (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/ Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as, for example, soya oil, sunflower oil, groundnut oil and coconut fat, fatty acids, such as, for example, palmitic acid, stearic acid and linoleic acid, alcohols, such as, for example, glycerol and ethanol, and organic acids, such as, for example, acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as, for example, magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as, for example, fatty acid polyglycol esters, can be employed to control the development of foam. Suitable substances having a selective action, such as, for example, antibiotics, can be added to the medium to maintain the stability of plasmids. To maintain aerobic conditions, oxygen or oxygen-containing gas mixtures, such as, for example, air, are introduced into the culture. The temperature of the culture is usually 20°C to 45°C, and preferably 25°C to 40°C. Culturing is continued until a maximum of the desired product has formed. This target is usually reached within 10 hours to 160 hours.

The fermentation broths obtained in this way, in particular containing L-methionine, usually have a dry weight of 7.5 to 25 wt.% and contain L-methionine. It is furthermore also advantageous if the fermentation is conducted in a sugar-limited procedure at least at the end, but in particular over at least 30% of the duration of the fermentation. That is to say, the concentration of utilizable sugar in the fermentation medium is reduced to ≥ 0 to 3 g/l during this period.

The fermentation broth prepared in this manner, in particular containing L-methionine, is then further processed. Depending on requirements, all or some of the biomass can be removed from the fermentation broth by separation methods, such as e.g. centrifugation, filtration, decanting or a combination thereof, or it can be left completely in this. This broth is then thickened or concentrated by known methods, such as e.g. with the aid of a rotary evaporator, thin film evaporator, falling film evaporator, by reverse osmosis, or by nanofiltration. This concentrated fermentation broth can then be worked up by methods of freeze drying, spray drying, spray granulation or by other processes to give a preferably free-flowing, finely divided powder.

This free-flowing, finely divided powder can then in turn by converted by suitable compacting or granulating processes into a coarse-grained, readily free-flowing, storable and largely dust-free product. In the granulation or compacting it is advantageous to employ conventional organic or inorganic auxiliary substances or carriers, such as starch, gelatin, cellulose derivatives or similar substances, such as are conventionally used as binders, gelling agents or thickeners in foodstuffs or feedstuffs processing, or further substances, such as, for example, silicas, silicates or stearates.

"Free-flowing" is understood as meaning powders which flow unimpeded out of the vessel with the opening of 5 mm (millimeters) of a series of glass outflow vessels with outflow openings of various sizes (Klein, Seifen, Öle, Fette, Wachse 94, 12 (1968)).

As described here, "finely divided" means a powder with a predominant content (> 50 %) with a particle size of 20 to 200 µm diameter. "Coarse-grained" means products with a predominant content (> 50 %) with a particle size of 200 to 2000 µm diameter. In this context, "dust-free" means that the product contains only small contents (< 5 %) with particle sizes of less than 20 µm diameter. The particle size determination can be carried out with methods of laser diffraction spectrometry. The corresponding methods are described in the textbook on "Teilchengrößenmessung in der Laborpraxis" by R. H. Müller and R. Schuhmann, Wissenschaftliche Verlagsgesellschaft Stuttgart (1996) or in the textbook "Introduction to Particle Technology" by M. Rhodes, Verlag Wiley & Sons (1998).

"Storable" in the context of this invention means a product which can be stored for up to 120 days, preferably up to 52 weeks, particularly preferably 60 months, without a substantial loss (< 5%) of methionine occurring.

Alternatively, however, the product can be absorbed on to an organic or inorganic carrier substance which is known and conventional in feedstuffs processing, such as, for example, silicas, silicates, grits, brans, meals, starches, sugars or others, and/or mixed and stabilized with conventional thickeners or binders. Use examples and processes in this context are described in the literature (Die Mühle + Mischfuttertechnik 132 (1995) 49, page 817).

Finally, the product can be brought into a state in which it is stable to digestion by animal stomachs, in particular the stomach of ruminants, by coating processes ("coating") using film-forming agents, such as, for example, metal carbonates, silicas, silicates, alginates, stearates, starches, gums and cellulose ethers, as described in DE-C-4100920.

If the biomass is separated off during the process, further inorganic solids, for example added during the fermentation, are in general removed. In addition, the animal feedstuffs additive according to the invention comprises at least the predominant proportion of the further substances, in particular organic substances, which are formed or added and are present in solution in the fermentation broth, where these have not been separated off by suitable processes.

In one aspect of the invention, the biomass can be separate off to the extent of up to 70%, preferably up to 80%, preferably up to 90%, preferably up to 95%, and particularly preferably up to 100%. In another aspect of the invention, up to 20% of the biomass, preferably up to 15%, preferably up to 10%, preferably up to 5%, particularly preferably no biomass is separated off.

These organic substances include organic by-products which are optionally produced, in addition to the L-methionine, and optionally discharged by the microorganisms employed in the fermentation. These include L-amino acids chosen from the group consisting of L-valine, L-threonine, L-alanine or L-tryptophan. They include vitamins chosen from the group consisting of vitamin B1 (thiamine), vitamin B2 (riboflavin),vitamin B5 (pantothenic acid), vitamin B6 (pyridoxine), vitamin B12 (cyanocobalamin), nicotinic acid/nicotinamide and vitamin E (tocopherol). They include furthermore organic acids which carry one to three carboxyl groups, such as, for example, acetic acid, lactic acid, citric acid, malic acid or fumaric acid. Finally, they also include sugars, such as, for example, trehalose. These compounds are optionally desired if they improve the nutritional value of the product.

These organic substances, including L-methionine and/or D-methionine and/or the racemic mixture D,L-methionine, can also be added, depending on requirements, as a concentrate or pure substance in solid or liquid form during a suitable process step. These organic substances mentioned can be added individually or as mixtures to the resulting or concentrated fermentation broth, or also during the drying or granulation process. It is likewise possible to add an organic substance or a mixture of several organic substances to the fermentation broth and a further organic substance or a further mixture of several organic substances during a later process step, for example granulation.

The product described above is suitable as a feedstuffs additive, i.e. feed additive, for animal nutrition.

The L-methionine content of the animal feedstuffs additive is conventionally 1 wt.% to 80 wt.%, preferably 2 wt.% to 80 wt.%, particularly preferably 4 wt.% to 80 wt.%, and very particularly preferably 8 wt.% to 80 wt.%, based on the dry weight of the animal feedstuffs additive. Contents of 1 wt.% to 60 wt.%, 2 wt.% to 60 wt.%, 4 wt.% to 60 wt.%, 6 wt.% to 60 wt.%, 1 wt.% to 40 wt.%, 2 wt.% to 40 wt.% or 4 wt.% to 40 wt.% are likewise possible. The water content of the feedstuffs additive is conventionally up to 5 wt.%, preferably up to 4 wt.%, and particularly preferably less than 2 wt.%.

An animal feedstuffs additive according to the present invention can comprise 1 wt.% to 80 wt.% L-methionine, D-methionine, D,L-methionine, or a mixture thereof with 1 to 40 wt.% L-lysine, D-lysine or D,L-lysine, based on the dry weight of the animal feedstuffs additive.

The invention accordingly also provides a process for the preparation of an L-methionine-containing animal feedstuffs additive from fermentation broths, which comprises the steps
a) culture and fermentation of an L-methionine-producing microorganism in a fermentation medium;
b) removal of water from the L-methionine-containing fermentation broth (concentration);
c) removal of an amount of 0 to 100 wt.% of the biomass formed during the fermentation; and
d) drying of the fermentation broth obtained according to a) and/or b) to obtain the animal feedstuffs additive in the desired powder or granule form.

If desired, one or more of the following steps can furthermore be carried out in the process according to the invention:
e) addition of one or more organic substances, including L-methionine and/or D-methionine and/or the racemic mixture D,L-methionine, to the products obtained according to a), b) and/or c);
f) addition of auxiliary substances chosen from the group consisting of silicas, silicates, stearates, grits and bran to the substances obtained according to a) to d) for stabilization and to increase the storability; or
g) conversion of the substances obtained according to a) to e) into a form stable to the animal stomach, in particular rumen, by coating with film-forming agents.

The analysis of L-methionine can be carried out by ion exchange chromatography with subsequent ninhydrin derivation, as described by Spackman et al. (Analytical Chemistry, 30, (1958), 1190).

Methods for the determination of L-amino acids are known from the prior art. The analysis can thus be carried out, for example, as described by Spackman et al. (Analytical Chemistry, 30, (195-8), 1190) by anion exchange chromatography with subsequent ninhydrin derivation, or it can be carried out by reversed phase HPLC, for example as described by Lindroth et al. (Analytical Chemistry (1979) 51: 1167-1174).

The process according to the invention is used for fermentative preparation of amino acids.

The present invention is explained in more detail in the following with the aid of embodiment examples.

The isolation of plasmid DNA from Escherichia coli and all techniques of restriction, Klenow and alkaline phosphatase treatment were carried out by the method of Sambrook et al. (Molecular Cloning. A Laboratory Manual, 1989, Cold Spring Harbor Laboratory Press, Cold Spring Harbor, NY, USA). Methods for transformation of Escherichia coli are also described in this handbook.

The composition of the usual nutrient media, such as LB or TY medium, can also be found in the handbook by Sambrook et al.

### Example 1

### Preparation of a genomic cosmid gene library from C. glutamicum ATCC 13032

Chromosomal DNA from C. glutamicum ATCC 13032 is isolated as described by Tauch et al. (1995, Plasmid 33:168-179) and partly cleaved with the restriction enzyme Sau3AI (Amersham Pharmacia, Freiburg, Germany, Product Description Sau3AI, Code no. 27-0913-02). The DNA fragments are dephosphorylated with shrimp alkaline phosphatase (Roche Molecular Biochemicals, Mannheim, Germany, Product Description SAP, Code no. 1758250). The DNA of the cosmid vector SuperCos1 (Wahl et al. (1987), Proceedings of the National Academy of Sciences, USA 84:2160-2164), obtained from Stratagene (La Jolla, USA, Product Description Supercos1 Cosmid Vector Kit, Code no. 251301) is cleaved with the restriction enzyme XbaI (Amersham Pharmacia, Freiburg, Germany, Product Description XbaI, Code no. 27-0948-02) and likewise dephosphorylated with shrimp alkaline phosphatase.

The cosmid DNA is then cleaved with the restriction enzyme BamHI (Amersham Pharmacia, Freiburg, Germany, Product Description BamHI, Code no. 27-0868-04). The cosmid DNA treated in this manner is mixed with the treated ATCC13032 DNA and the batch is treated with T4 DNA ligase (Amersham Pharmacia, Freiburg, Germany, Product Description T4-DNA-Ligase, Code no.27-0870-04). The ligation mixture is then packed in phages with the aid of Gigapack II XL Packing Extract (Stratagene, La Jolla, USA, Product Description Gigapack II XL Packing Extract, Code no. 200217).

For infection of the E. coli strain NM554 (Raleigh et al. 1988, Nucleic Acid Res. 16:1563-1575) the cells are taken up in 10 mum MgSO₄ and mixed with an aliquot of the phage suspension. The infection and titering of the cosmid library are carried out as described by Sambrook et al. (1989, Molecular Cloning: A Laboratory Manual, Cold Spring Harbor), the cells being plated out on LB agar (Lennox, 1955, Virology, 1:190) + 100 mg/l ampicillin. After incubation overnight at 37°C, recombinant individual clones are selected.

### Example 2

### Isolation and sequencing of the metD gene

The cosmid DNA of an individual colony is isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments are dephosphorylated with shrimp alkaline phosphatase (Roche Molecular Biochemicals, Mannheim, Germany, Product Description SAP, Product No. 1758250). After separation by gel electrophoresis, the cosmid fragments in the size range of 1500 to 2000 bp are isolated with the QiaExII Gel Extraction Kit (Product No. 20021, Qiagen, Hilden, Germany).

The DNA of the sequencing vector pZero-1, obtained from Invitrogen (Groningen, Holland, Product Description Zero Background Cloning Kit, Product No. K2500-01) is cleaved with the restriction enzyme BamHI, (Amersham Pharmacia, Freiburg, Germany, Product Description BamHI, Product No. 27-0868-04). The ligation of the cosmid fragments in the sequencing vector pZero-1 is carried out as described by Sambrook et al. (1989, Molecular Cloning: A Laboratory Manual, Cold Spring Harbor), the DNA mixture being incubated overnight with T4 ligase (Pharmacia Biotech, Freiburg, Germany). This ligation mixture is then electroporated (Tauch et al. 1994, FEMS Microbiol. Letters, 123:343-7) into the E. coli strain DH5αmcr (Grant, 1990, Proceedings of the National Academy of Sciences, U.S.A., 87:4645-4649) and plated out on LB agar (Lennox, 1955, Virology, 1:190) with 50 mg/l zeocin.

The plasmid preparation of the recombinant clones is carried out with a Biorobot 9600 (Product No. 900200, Qiagen, Hilden, Germany). The sequencing is carried out by the dideoxy chain-stopping method of Sanger et al. (1977, Proceedings of the National Academies of Sciences, U.S.A., 74:5463-5467) with modifications according to Zimmermann et al. (1990, Nucleic Acids Research, 18:1067). The "RR dRhodamin Terminator Cycle Sequencing Kit" from PE Applied Biosystems (Product No. 403044, Weiterstadt, Germany) was used. The separation by gel electrophoresis and analysis of the sequencing reaction are carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained are then processed using the Staden program package (1986, Nucleic Acids Research, 14:217-231) version 97-0. The individual sequences of the pzerol derivatives are assembled to a continuous contig. The computer-assisted coding region analysis is prepared with the XNIP program (Staden, 1986, Nucleic Acids Research 14:217-231). Further analyses are carried out with the "BLAST search programs" (Altschul et al., 1997, Nucleic Acids Research, 25:3389-3402) against the non-redundant databank of the "National Center for Biotechnology Information" (NCBI, Bethesda, MD, USA).

The resulting nucleotide sequence is shown in SEQ ID No. 1. Analysis of the nucleotide sequence shows an open reading frame of 711 bp, which is called the metD gene. The metD gene codes for a polypeptide of 236 amino acids.

The DNA sections lying upstream and downstream of SEQ ID No. 1 were identified in the same way, these sections being shown in SEQ ID No. 3 and SEQ ID No. 4. The metD gene region extended by SEQ ID No. 3 and SEQ ID No. 4 is shown in SEQ ID No. 5.

### Example 3

### Incorporation of a deletion into the metD gene

For this, chromosomal DNA is isolated from the strain ATCC13032 by the method of Tauch et al. (Plasmid 33:168-179 (1995)). On the basis of the sequence of the metD gene known for C. glutamicum from example 2, the oligonucleotides described below are chosen for generation of the metD deletion allele by means of the polymerase chain reaction (PCR) by the gene Soeing method (Horton, Molecular Biotechnology 3: 93-98 (1995)).
Primer nietD-DelA (see also SEQ ID No. 6):
   5' -GAT CTA AAG CTT-GCC TCT CCA ATC TCC ACT GA -3'
Primer metD-DelB (see also SEQ ID No. 7):
Primer metD-DelC (see also SEQ ID No. 8):
Primer metD-DelD (see also SEQ ID No. 9):
   5'-GAT CTA AAG CTT-GAT GTC CAT GTA CCG CAG C -3'

The primers shown are synthesized by MWG Biotech (Ebersberg, Germany) and the PCR reaction is carried out using Pfu polymerase (Stratagene, Product. No. 600135, La Jolla, USA) and a PTC 100 Thermocycler (MJ Research Inc., Waltham, USA).

The primers metD-DelA and metD-DelD contain in each case an inserted cleavage site for the restriction enzyme HindIII, and the primers metD-DelB and metD-DelC an inserted cleavage site for the restriction enzyme SwaI, which are marked by underlining in the nucleotide sequence shown above.

The primer metD-DelB is composed of two regions of the nucleotide sequence, one of which bonds in the coding sequence of metD to the nucleotides 707 to 688, and the other bonds to the "upstream region in front of the start codon of metD. Both regions are divided by the inserted SwaI restriction enzyme site. The Primer metD-DelC is reverse complementary to the Primer metD-DelB.

With the aid of the polymerase chain reaction the primers metD-DelA and metD-DelB enable the amplification of a 573-bp DNA fragment and the Primers metD-DelC and metD-DelD enable the amplification of a 651 bp DNA fragment. The amplificates are examined by subsequent agarose-gel electrophoresis in an 0,8% agarose-gel, isolated from the agarose-gel with the High Pure PCR Product Purification Kit (Product No. 1732676, Roche Diagnostics GmbH, Mannheim, Deutschland), and used together as a DNA template in another PCR reaction using the primers metD-DelA and metD-DelD. This results in the production of the metD deletion derivative, 1177 bp in size (see also SEQ ID No. 10).

The amplified product is subsequently examined in a 0,8% agarose-gel.

### Example 4

### 4.1 Construction of the exchange vector pK18mobsacBmetDdel

The metD deletion derivative obtained in example 3 is cleaved with the restriction endonuclease HindIII, after examination in a 0,8% agarose-gel isolated from the agarose gel with the High Pure PCR Product purification Kit (Product No. 1732676, Roche Diagnostics GmbH, Mannheim, Deutschland) and used for ligation with the mobilizable cloning vector pK18mobsacB described by Schäfer et al., Gene, 14, 69-73 (1994). The vector pK18mobsacB was cleaved beforehand with the restriction enzyme HindIII and subsequently dephosphorylated with shrimp alkaline phosphatase (Roche Diagnostics GmbH, Mannheim, Germany, Product Description SAP, Product No. 1758250). The prepared vector is then mixed with the metD deletion derivative and treated with T4 DNA ligase (Amersham-Pharmacia, Freiburg, Germany).

The E. coli strain DH5αmcr (Grant, 1990, Proceedings of the National Academy of Sciences U.S.A., 87: 4645-4649) is then electroporated with the ligation batch (Hanahan, In. DNA cloning. A practical approach. Vol.1. ILR-Press, Cold Spring Harbor, New York, 1989). Selection of plasmid-carrying cells is made by plating out the transformation batch on LB agar (Sambrook et al., Molecular Cloning: A Laboratory Manual, 2nd Ed., Cold Spring Harbor, New York, 1989), which has been supplemented with 25 mg/l kanamycin.

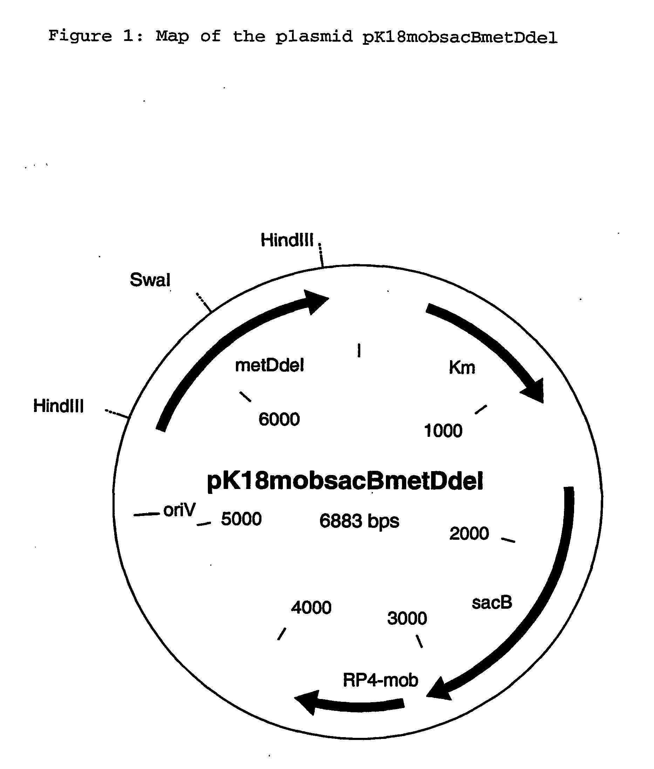

Plasmid DNA is isolated from a transformant with the aid of the QIAprep Spin Miniprep Kit from Qiagen and verified by cutting with the restriction enzymes HindIII and swaI. The plasmid is called pK18mobsacBmetDdel and is shown in figure 1. The strain is called E. coli DHSαmcr/pK18mobsacBmetDdel.

### 4.2 . Deletion mutagenesis of the metD gene in the C. glutamicum strain ATCC13032

The vector pK18mobsacBmetDdel mentioned in example 4.1 is electroporated by the electroporation method of Tauch et al.,(1989 FEMS Microbiology Letters 123: 343-347) in the strain C. glutamicum ATCC13032. The vector cannot replicate independently in ATCC13032 and is retained in the cell only if it has integrated into the chromosome. Selection of clones with integrated pK18mobsacmetDdel is carried out by plating out the electroporation batch on LB agar (Sambrook et al., Molecular Cloning: A Laboratory Manual, 2nd Ed., Cold Spring Harbor, New York, 1989), which has been supplemented with 15 mg/l kanamycin. Clones which had grown on are plated out on LB agar plates with 25 mg/l kanamycin and incubated for 16 hours at 33°C.

To achieve excision of the plasmid together with the complete chromosomal copy of the metD gene, the clones are incubated unselectively overnight in LB medium and then cultured on LB agar with 10% sucrose. The plasmid pK18mobsacB contains a copy of the sacB gene, which converts sucrose into levan sucrose, which is toxic to C. glutamicum. Only those clones in which the integrated pK18mobsacBmetDdel has been excised again therefore grow on LB agar with sucrose. In the excision, together with the plasmid either the complete chromosomal copy of the metD gene can be excised, or the metD deletion derivative.

To demonstrate that the metD gene is deleted in the chromosome, the plasmid pK18mobsacBmetDdel is marked by the method of "The DIG System Users Guide for Filter Hybridization" of Boehringer Mannheim GmbH (Mannheim, Germany, 1993) using the DIG hybridization kit from Boehringer. Chromosomal DNA of a potential deletion mutant is isolated by the method of Eikmanns et al. (Microbiology 140: 1817-1828 (1994)) and in each case cleaved with the restriction enzymes HindIII and SwaI in separate batches. The fragments formed are separated by agarose gel electrophoresis and hybridized at 68°C with the Dig hybridization kit from Boehringer. With the aid of the fragments formed, it can be shown that the strain ATCC13032 has lost its copy of the metD gene, and instead carries the deleted allele.

The strain is called C. glutamicum ATCC13032AmetD.

### Example 5

### Production of methionine

The C. glutamicum strain ATCC13032AmetD obtained in example 4 is cultured in a nutrient medium suitable for the production of methionine and the methionine content in the culture supernatant is determined.

For this, the strain is first incubated on a brain-heart agar plate for 24 hours at 33°C. Starting from this agar plate culture, a preculture is seeded (10 ml medium in a 100 ml conical flask). The medium MM is used as the medium for the preculture.

| Medium MM | |
|---|---|
| CSL (corn steep liquor) | 5 g/l |
| MOPS (morpholinopropanesulfonic acid) | 20 g/l |
| Glucose (autoclaved separately) | 50g/l |
| Salts: | |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.01 mg/l |
| Vitamin B12 (sterile-filtered) | 0.02 mg/l |
| Thiamine * HCl (sterile-filtered) | 0.2 mg/l |
| CaCO₃ | 25 g/l |

The CSL, MOPS and the salt solution are brought to pH 7 with aqueous ammonia and autoclaved. The sterile substrate and vitamin solutions are then added, as well as the CaCO₃ autoclaved in the dry state.

The preculture is incubated for 16 hours at 33°C at 240 rpm on a shaking machine. A main culture is seeded from this preculture such that the initial OD (660 nm) of the main culture was 0.1. Medium MM is also used for the main culture.

Culturing is carried out in a 10 ml volume in a 100 ml conical flask with baffles. Culturing is carried out at 33°C and 80% atmospheric humidity.

After 72 hours, the OD is determined at a measurement wavelength of 660 nm with a Biomek 1000 (Beckmann Instruments GmbH, Munich). The amount of methionine formed is determined with an amino acid analyzer from Eppendorf-BioTronik (Hamburg, Germany) by ion exchange chromatography and post-column derivation with ninhydrin detection.

The result of the experiment is shown in Table 1.

**Table 1**

| Strain | OD (660 nm) | Methionine mg/l |
|---|---|---|
| ATCC13032 | 12.2 | 3 |
| ATCC13032ΔmetD | 14.8 | 20 |

### Brief Description of the Figure:

Figure 1: Map of the plasmid pK18mobsacBmetDdel

The abbreviations and designations used have the following meaning:
- oriv: ColE1-similar origin from pMB1
- sacB: sacB gene coding for the protein levansucrase
- KmR: Kanamycin resistance
- HindIII: Cleavage site of the restriction enzyme HindIII
- SwaI: Cleavage site of the restriction enzyme SwaI
- RP4mob: RP4 mobilization site
- metDdel: Cloned deletion derivative for metD

## Claims

1. Coryneform bacterium in which the metD gene is attenuated, wherein the metD gene comprises a polynucleotide which
a) is identical to the extent of at least 70% to a polynucleotide which codes for a polypeptide which comprises the amino acid sequence of SEQ ID No. 2, or
b) codes for a polypeptide which comprises an amino acid sequence which is identical to the extent of at least 70% to the amino acid sequence of SEQ ID No. 2,
and the polypeptide has transcription regulator activity
wherein attenuation is achieved by
- using a weak promoter,
- using an allele which codes for a protein with low activity or
- inactivating the gene.

2. Coryneform bacterium according to claim 1 in which the metD gene is eliminated.

3. Coryneform bacterium according to claims 1 or 2 in which the polynucleotide is a DNA which is capable of replication, comprising
(i) the nucleotide sequence shown in SEQ ID No.1, or
(ii) at least one sequence which corresponds to sequence (i) within the range of the degeneration of the genetic code, or
(iii) at least one sequence which hybridizes with the sequence complementary to sequence (i) or (ii) under a stringency corresponding to at most 2x SSC and optionally
(iv) sense mutations of neutral function in (i).

4. Coryneform bacterium according to claim 1, wherein said polynucleotide codes for a polypeptide which comprises the amino acid sequence shown in SEQ ID No. 2.

5. Coryneform bacteria according to claims 1 to 4, wherein said inactivation is achieved by a vector which carries at least 15 succssive nucleotides of the sequence shown in SEQ ID NO. 1

6. Coryneform bacteria according to one or more of claims 1 to 5, wherein said bacteria are of the species Corynebacterium glutamicum.

7. Process for the preparation of L-amino acids, wherein the following steps are carried out:
a) fermentation of the coryneform bacteria according to one of claims 1 to 6 which produce the desired L-amino acid;
b) concentration of the L-amino acid in the medium or in the cells of the bacteria, and
c) isolation of the L-amino acid.

8. Process according to claim 7, wherein the L-amino acid is L-methionine.

9. A process for the preparation of an L-methionine-containing animal feedstuffs additive from fermentation broths, which comprises the steps
a) culture and fermentation of an L-methionine-producing microorganism according to claims 1 to 6 in a fermentation medium;
b) removal of water from the L-methionine-containing fermentation broth (concentration);
c) removal of an amount of 0 to 100 wt.% of the biomass formed during the fermentation; and
d) drying of the fermentation broth obtained according to b) and/or c) to obtain the animal feedstuffs additive in the desired powder or granule form.

10. A process according to claim 9, wherein one or more of the following steps is or are additionally carried out:
e) addition of one or more organic substances, including L-methionine and/or D-methionine and/or the racemic mixture D,L-methionine, to the products obtained according to b), c) and/or d);
f) addition of auxiliary substances chosen from the group consisting of silicas, silicates, stearates, grits and bran to the substances obtained according to b) to e) for stabilization and to increase the storability; or
g) conversion of the substances obtained according to b) to f) into a form stable to the animal stomach, in particular rumen, by coating with film-forming agents.

11. A process according to claims 9 or 10, wherein up to 100% of the biomass is removed.

12. A process according to claim 9 or 10, wherein the water content is up to 5 wt.%.

13. A process as claimed in claims 10, 11 or 12, wherein the film-forming agents are metal carbonates, silicas, silicates, alginates, stearates, starches, gums or cellulose ethers.

## Patentansprüche

1. Coryneformes Bakterium, in dem das metD-Gen abgeschwächt ist, wobei das metD-Gen ein Polynukleotid umfasst, das
a) in einem Ausmaß von mindestens 70% identisch zu einem Polynukleotid ist, das für ein Polypeptid kodiert, das die Aminosäuresequenz der SEQ ID NR: 2 umfasst, oder
b) für ein Polypeptid kodiert, das eine Aminosäuresequenz umfasst, die in einem Ausmaß von mindestens 70% identisch zu der Aminosäuresequenz der SEQ ID NR: 2 ist,
und das Polypeptid Transkriptionsregulator-Aktivität hat,
wobei die Abschwächung **dadurch** erzielt wird, dass man
- einen schwachen Promotor verwendet,
- ein Allel verwendet, das für ein Protein mit niedriger Aktivität kodiert, oder
- das Gen inaktiviert.

2. Coryneformes Bakterium nach Anspruch 1, wobei das metD-Gen beseitigt ist.

3. Coryneformes Bakterium nach den Ansprüchen 1 oder 2, wobei das Polynukleotid eine DNA ist, die zur Replikation in der Lage ist, umfassend
(i) die in SEQ ID NR: 1 dargestellte Nukleotidsequenz oder
(ii) mindestens eine Sequenz, die der Sequenz (i) im Rahmen der Degeneration des genetischen Codes entspricht, oder
(iii) mindestens eine Sequenz, die mit der Sequenz, die komplementär zu der Sequenz (i) oder (ii) ist, unter einer Stringenz hybridisiert, die höchstens 2X SSC entspricht, und gegebenenfalls
(iv) Sense-Mutationen mit neutraler Funktion in (i).

4. Coryneformes Bakterium nach Anspruch 1, wobei das Polynukleotid ein Polypeptid kodiert, das die in SEQ ID NR: 2 dargestellte Aminosäuresequenz umfasst.

5. Coryneforme Bakterien nach den Ansprüchen 1 bis 4, wobei die Inaktivierung durch einen Vektor erzielt wird, der mindestens 15 aufeinander folgende Nukleotide der in SEQ ID NR: 1 dargestellten Sequenz trägt.

6. Coryneforme Bakterien nach einem oder mehreren der Ansprüche 1 bis 5, wobei die Bakterien aus der Spezies Corynebacterium glutamicum sind.

7. Verfahren zur Herstellung von L-Aminosäuren, wobei man die folgenden Schritte durchführt:
a) Fermentation der coryneformen Bakterien nach einem der Ansprüche 1 bis 6, die die gewünschte L-Aminosäure produzieren;
b) Aufkonzentrieren der L-Aminosäure in dem Medium oder den Zellen der Bakterien und
c) Isolation der L-Aminosäure.

8. Verfahren nach Anspruch 7, wobei die L-Aminosäure L-Methionin ist.

9. Verfahren zur Herstellung eines L-Methionin enthaltenden Tierfutteradditivs aus Fermentationsbrühen, das folgende Schritte umfasst:
a) Kultur und Fermentation eines L-Methionin produzierenden Mikroorganismus nach den Ansprüchen 1 bis 6 in einem Fermentationsmedium;
b) Entfernen von Wasser aus der L-Methionin enthaltenden Fermentationsbrühe (Einengen);
c) Entnehmen einer Menge von 0 bis 100 Gew.-% der während der Fermentation gebildeten Biomasse und
d) Trocknen der gemäß b) und/oder c) erhaltenen Fermentationsbrühe, um das Tierfutteradditiv in der gewünschten Pulver- oder Granulatform zu erhalten.

10. Verfahren nach Anspruch 9, wobei zusätzlich einer oder mehrere der folgenden Schritte ausgeführt wird/werden:
e) Zugabe von einer oder mehreren organischen Substanzen, einschließlich L-Methionin und/oder D-Methionin und/oder des racemischen Gemischs D,L-Methionin, zu den gemäß b), c) und/oder d) erhaltenen Produkten;
f) Zugabe von Hilfssubstanzen, die aus der Gruppe ausgewählt werden, bestehend aus Kieselerden, Silikaten, Stearaten, Gries und Kleie, zu den gemäß b) bis e) erhaltenen Substanzen zur Stabilisierung und zur Steigerung der Lagerungsfähigkeit; oder
g) Umwandlung der gemäß b) bis f) erhaltenen Substanzen in eine Form, die für den Tiermagen, insbesondere den Pansen (Rumen), stabil ist, durch Beschichten mit filmbildenden Mitteln.

11. Verfahren nach den Ansprüchen 9 oder 10, wobei bis zu 100% der Biomasse entfernt werden.

12. Verfahren nach Anspruch 9 oder 10, wobei der Wassergehalt bis zu 5 Gew.-% beträgt.

13. Verfahren nach den Ansprüchen 10, 11 oder 12, wobei es sich bei den filmbildenden Mitteln um Metallcarbonate, Kieselerden, Silikate, Alginate, Stearate, Stärken, Gummen oder Celluloseether handelt.

## Revendications

1. Bactérie corynéforme dans laquelle le gène metD est atténué, où le gène metD comprend un polynucléotide qui
a) est identique à un degré d'au moins 70 % à un polynucléotide qui code pour un polypeptide qui comprend la séquence d'acides aminés de SEQ ID N° 2, ou
b) code pour un polypeptide qui comprend une séquence d'acides aminés qui est identique à un degré d'au moins 70 % à la séquence d'acides aminés de SEQ ID N° 2,
et le polypeptide a une activité régulatrice de transcription
où l'atténuation est obtenue par
- utilisation d'un promoteur faible,
- utilisation d'un allèle qui code pour une protéine à faible activité ou
- inactivation du gène.

2. Bactérie corynéforme selon la revendication 1 dans laquelle le gène metD est éliminé.

3. Bactérie corynéforme selon la revendication 1 ou 2, dans laquelle le polynucléotide est un ADN qui est capable de réplication, comprenant
(i) la séquence nucléotidique décrite dans SEQ ID N° 1, ou
(ii) au moins une séquence qui correspond à la séquence (i) dans les limites de la dégénérescence du code génétique, ou
(iii) au moins une séquence qui s'hybride avec la séquence complémentaire de la séquence (i) ou (ii) sous une stringence correspondant au plus à 2x SSC, et éventuellement
(iv) des mutations sens de fonction neutre dans (i).

4. Bactérie corynéforme selon la revendication 1, dans laquelle ledit polynucléotide code pour un polypeptide qui comprend la séquence d'acides aminés décrite dans SEQ ID N° 2.

5. Bactérie corynéforme selon les revendications 1 à 4, dans laquelle ladite inactivation est effectuée par un vecteur qui comporte au moins 15 nucléotides successifs de la séquence décrite dans SEQ ID N° 1.

6. Bactérie corynéforme selon une ou plusieurs des revendications 1 à 5, dans laquelle lesdites bactéries sont de l'espèce Corynebacterium glutamicum.

7. Procédé pour la préparation d'acides aminés L, dans lequel les étapes suivantes sont conduites :
a) fermentation des bactéries corynéformes selon une des revendications 1 à 6 qui produisent l'acide aminé L ;
b) concentration de l'acide aminé L dans le milieu ou dans les cellules des bactéries, et
c) isolement de l'acide aminé L.

8. Procédé selon la revendication 7, dans lequel l'acide aminé L est la L-méthionine.

9. Procédé pour la préparation d'un additif pour aliments pour animaux contenant de la L-méthionine à partir de bouillons de fermentation, qui comprend les étapes de
a) culture et fermentation d'un micro-organisme produisant de la L-méthionine selon les revendications 1 à 6 dans un milieu de fermentation ;
b) élimination de l'eau du bouillon de fermentation contenant de la L-méthionine (concentration) ;
c) élimination d'une quantité de 0 à 100 % en poids de la biomasse formée pendant la fermentation ; et
d) séchage du bouillon de fermentation obtenu selon b) et/ou c) pour obtenir l'additif pour aliments pour animaux sous la forme souhaitée de poudre ou de granule.

10. Procédé selon la revendication 9, dans lequel une ou plusieurs des étapes suivantes est ou sont en outre conduites :
e) ajout d'une ou plusieurs substances organiques, comprenant de la L-méthionine, et/ou de la D-méthionine et/ou le mélange racémique de D,L-méthionine, aux produits obtenus selon b), c) et/ou d) ;
f) ajout de substances auxiliaires choisies dans le groupe constitué de silices, silicates, stéarates, gruau et son aux substances obtenues selon b) à e) pour stabilisation et pour augmenter l'aptitude au stockage ; ou
g) conversion des substances obtenues selon b) à f) sous une forme stable dans l'estomac de l'animal, en particulier le rumen, par enrobage avec des agents pelliculants.

11. Procédé selon la revendication 9 ou 10, dans lequel jusqu'à 100 % de la biomasse sont éliminés.

12. Procédé selon la revendication 9 ou 10, dans lequel la teneur en eau est de jusqu'à 5 % en poids.

13. Procédé selon la revendication 10, 11 ou 12, dans lequel les agents filmogènes sont des carbonates de métal, des silices, des silicates, des alginates, des stéarates, des amidons, des gommes ou des éthers de cellulose.


### Sequence Listing

<110> Degussa AG
<120> Nucleotide Sequences which Code for the metD Gene
<130> 010191 BT
<160> 11
<170> PatentIn version 3.1
<210> 1
   <211> 1322
   <212> DNA
   <213> Corynebacterium glutamicum
<220>
   <221> CDS
   <222> (314)..(1021)
   <223> metD-Gen
<400> 1
   agattaaagg cactgatgct cagcaaggaa ttttgctgaa catagtcgtc ggtattatcg      60
   gtggtttgtt aggcggctgg ctgcttggaa tcttcggagt ggatgttgcc ggtggcggct     120
   tgatcttcag cttcatcaca tgtctgattg gtgctgtcat tttgctgacg atcgtgcagt     180
   tcttcactcg gaagaagtaa tctgctttaa atccgtaggg cctgttgata tttcgatatc     240
   aacaggcctt ttggtcattt tggggtggaa aaagcgctag acttgcctgt ggattaaaac     300
   tatacgaacc ggt ttg tct ata ttg gtg tta gac agt tcg tcg tat ctt        349
                  Met Ser Ile Leu Val Leu Asp Ser Ser Ser Tyr Leu
                  1               5                   10
   gaa aca gac caa ccc gaa agg acg tgg ccg aac gtg gct gct agc gct       397
   Glu Thr Asp Gln Pro Glu Arg Thr Trp Pro Asn Val Ala Ala Ser Ala
           15                  20                  25
   tca ggc aag agt aaa aca agt gcc ggg gca aac cgt cgt cgc aat cga       445
   Ser Gly Lys Ser Lys Thr Ser Ala Gly Ala Asn Arg Arg Arg Asn Arg
       30                  35                  40
   cca agc ccc cga cag cgt ctc ctc gat agc gca acc aac ctt ttc acc       493
   Pro Ser Pro Arg Gln Arg Leu Leu Asp Ser Ala Thr Asn Leu Phe Thr
   45                  50                  55                  60
   aca gaa ggt att cgc gtc atc ggt att gat cgt atc ctc cgt gaa gct       541
   Thr Glu Gly Ile Arg Val Ile Gly Ile Asp Arg Ile Leu Arg Glu Ala
                   65                  70                  75
   gac gtg gcg aag gcg agc ctc tat tcc ctt ttc gga tcg aag gac gcc       589
   Asp Val Ala Lys Ala Ser Leu Tyr Ser Leu Phe Gly Ser Lys Asp Ala
               80                  85                  90
   ttg gtt att gca tac ctg gag aac ctc gat cag ctg tgg cgt gaa gcg       637
   Leu Val Ile Ala Tyr Leu Glu Asn Leu Asp Gln Leu Trp Arg Glu Ala
           95                  100                 105
   tgg cgt gag cgc acc gtc ggt atg aag gat ccg gaa gat aaa atc atc      685
   Trp Arg Glu Arg Thr Val Gly Met Lys Asp Pro Glu Asp Lys Ile Ile
       110                 115                 120
   gcg ttc ttt gat cag tgc att gag gaa gaa cca gaa aaa gat ttc cgc      733
   Ala Phe Phe Asp Gln Cys Ile Glu Glu Glu Pro Glu Lys Asp Phe Arg
   125                 130                 135                 140
   ggc tcg cac ttt cag aat gcg gct agt gag tac cct cgc ccc gaa act      781
   Gly Ser His Phe Gln Asn Ala Ala Ser Glu Tyr Pro Arg Pro Glu Thr
                   145                 150                 155
   gat agc gaa aag ggc att gtt gca gca gtg tta gag cac cgc gag tgg      829
   Asp Ser Glu Lys Gly Ile Val Ala Ala Val Leu Glu His Arg Glu Trp
               160                 165                 170
   tgt cat aag act ctg act gat ttg ctc act gag aag aac ggc tac cca      877
   Cys His Lys Thr Leu Thr Asp Leu Leu Thr Glu Lys Asn Gly Tyr Pro
           175                 180                 185
   ggc acc acc cag gcg aat cag ctg ttg gtg ttc ctt gat ggt gga ctt      925
   Gly Thr Thr Gln Ala Asn Gln Leu Leu Val Phe Leu Asp Gly Gly Leu
       190                 195                 200
   gct gga tct cga ttg gtc cac aac atc agt cct ctt gag acg gct cgc      973
   Ala Gly Ser Arg Leu Val His Asn Ile Ser Pro Leu Glu Thr Ala Arg
   205                 210                 215                 220
   gat ttg gct cgg cag ttg ttg tcg gct cca cct gcg gac tac tca att     1021
   Asp Leu Ala Arg Gln Leu Leu Ser Ala Pro Pro Ala Asp Tyr Ser Ile
                   225                 230                 235
   tagtttcttc attttccgaa ggggtatctt cgttggggga ggcgtcgata agccccttct   1081
   ttttagcttt aacctcagcg cgacgctgct ttaagcgctg catggcggcg cggttcattt   1141
   cacgttgcgt ttcgcgcctc ttgttcgcga tttctttgcg ggcctgtttt gcttcgttga   1201
   tttcggcagt acgggttttg gtgagttcca cgtttgttgc gtgaagcgtt gaggcgttcc   1261
   atggggtgag aatcatcagg gcgcggtttt tgcgtcgtgt ccacaggaag atgcgctttt   1321
   c                                                                   1322
<210> 2
   <211> 236
   <212> PRT
   <213> Corynebacterium glutamicum
<400> 2
   Met Ser Ile Leu Val Leu Asp Ser Ser Ser Tyr Leu Glu Thr Asp Gln
   1               5                   10                  15
   Pro Glu Arg Thr Trp Pro Asn Val Ala Ala Ser Ala Ser Gly Lys Ser
               20                  25                  30
   Lys Thr Ser Ala Gly Ala Asn Arg Arg Arg Asn Arg Pro Ser Pro Arg
           35                  40                  45
   Gln Arg Leu Leu Asp Ser Ala Thr Asn Leu Phe Thr Thr Glu Gly Ile
       50                  55                  60
   Arg Val Ile Gly Ile Asp Arg Ile Leu Arg Glu Ala Asp Val Ala Lys
   65                  70                  75                  80
   Ala Ser Leu Tyr Ser Leu Phe Gly Ser Lys Asp Ala Leu Val Ile Ala
                   85                  90                  95
   Tyr Leu Glu Asn Leu Asp Gln Leu Trp Arg Glu Ala Trp Arg Glu Arg
               100                 105                 110
   Thr Val Gly Met Lys Asp Pro Glu Asp Lys Ile Ile Ala Phe Phe Asp
           115                 120                 125
   Gln Cys Ile Glu Glu Glu Pro Glu Lys Asp Phe Arg Gly Ser His Phe
       130                 135                 140
   Gln Asn Ala Ala Ser Glu Tyr Pro Arg Pro Glu Thr Asp Ser Glu Lys
   145                 150                 155                 160
   Gly Ile Val Ala Ala Val Leu Glu His Arg Glu Trp Cys His Lys Thr
                   165                 170                 175
   Leu Thr Asp Leu Leu Thr Glu Lys Asn Gly Tyr Pro Gly Thr Thr Gln
               180                 185                 190
   Ala Asn Gln Leu Leu Val Phe Leu Asp Gly Gly Leu Ala Gly Ser Arg
           195                 200                 205
   Leu Val His Asn Ile Ser Pro Leu Glu Thr Ala Arg Asp Leu Ala Arg
       210                 215                 220
   Gln Leu Leu Ser Ala Pro Pro Ala Asp Tyr Ser Ile
   225                 230                 235
<210> 3
   <211> 239
   <212> DNA
   <213> Corynebacterium glutamicum
<220>
   <221> misc_feature
   <223> upstream-Bereich
<400> 3
   gcctctccaa tctccactga ggtacttaat ccttccgggg aattcgggcg cttaaatcga 60
   gaaattaggc catcaccttt taataacaat acaatgaata attggaatag gtcgacacct 120
   ttggagcgga gccggttaaa attggcagca ttcaccgaaa gaaaaggaga accacatgct 180
   tgccctaggt tggattacat ggatcattat tggtggtcta gctggttgga ttgcctcca 239
<210> 4
   <211> 289
   <212> DNA
   <213> Corynebacterium glutamicum
<220>
   <221> misc_feature
   <223> downstream-Bereich
<400> 4
   tttttgtttt gcgcggtaga tgtcgcgctg ctctaggtgg tgcactttga aatcgtcggt 60
   aagtgggtat ttgcgttcca aaatgaccat catgatgatt gtttggagga gcgtccacag 120
   gttgttgctg acccaataga gtgcgattgc tgtggggaat ggtcctgtga ggccaaggga 180
   cagtgggaag atcggcgcga ggatcgacat cacgatcatg aacttcagca tgccgttaga 240
   gaatccggat gcgtaatcgt tggtttggaa gctgcggtac atggacatc 289
<210> 5
   <211> 1850
   <212> DNA
   <213> Corynebacterium glutamicum
<220>
   <221> CDS
   <222> (553)..(1260)
   <223> metD-Gen
<400> 5
   gcctctccaa tctccactga ggtacttaat ccttccgggg aattcgggcg cttaaatcga     60
   gaaattaggc catcaccttt taataacaat acaatgaata attggaatag gtcgacacct    120
   ttggagcgga gccggttaaa attggcagca ttcaccgaaa gaaaaggaga accacatgct    180
   tgccctaggt tggattacat ggatcattat tggtggtcta gctggttgga ttgcctccaa    240
   gattaaaggc actgatgctc agcaaggaat tttgctgaac atagtcgtcg gtattatcgg    300
   tggtttgtta ggcggctggc tgcttggaat cttcggagtg gatgttgccg gtggcggctt    360
   gatcttcagc ttcatcacat gtctgattgg tgctgtcatt ttgctgacga tcgtgcagtt    420
   cttcactcgg aagaagtaat ctgctttaaa tccgtagggc ctgttgatat ttcgatatca    480
   acaggccttt tggtcatttt ggggtggaaa aagcgctaga cttgcctgtg gattaaaact    540
   atacgaaccg gt ttg tct ata ttg gtg tta gac agt tcg tcg tat ctt gaa    591
                 Met Ser Ile Leu Val Leu Asp Ser Ser Ser Tyr Leu Glu
                 1               5                   10
   aca gac caa ccc gaa agg acg tgg ccg aac gtg gct gct agc gct tca      639
   Thr Asp Gln Pro Glu Arg Thr Trp Pro Asn Val Ala Ala Ser Ala Ser
       15                  20                  25
   ggc aag agt aaa aca agt gcc ggg gca aac cgt cgt cgc aat cga cca      687
   Gly Lys Ser Lys Thr Ser Ala Gly Ala Asn Arg Arg Arg Asn Arg Pro
   30                  35                  40                  45
   agc ccc cga cag cgt ctc ctc gat agc gca acc aac ctt ttc acc aca      735
   Ser Pro Arg Gln Arg Leu Leu Asp Ser Ala Thr Asn Leu Phe Thr Thr
                   50                  55                  60
   gaa ggt att cgc gtc atc ggt att gat cgt atc ctc cgt gaa gct gac      783
   Glu Gly Ile Arg Val Ile Gly Ile Asp Arg Ile Leu Arg Glu Ala Asp
               65                  70                  75
   gtg gcg aag gcg agc ctc tat tcc ctt ttc gga tcg aag gac gcc ttg      831
   Val Ala Lys Ala Ser Leu Tyr Ser Leu Phe Gly Ser Lys Asp Ala Leu
           80                  85                  90
   gtt att gca tac ctg gag aac ctc gat cag ctg tgg cgt gaa gcg tgg      879
   Val Ile Ala Tyr Leu Glu Asn Leu Asp Gln Leu Trp Arg Glu Ala Trp
       95                  100                 105
   cgt gag cgc acc gtc ggt atg aag gat ccg gaa gat aaa atc atc gcg      927
   Arg Glu Arg Thr Val Gly Met Lys Asp Pro Glu Asp Lys Ile Ile Ala
   110                 115                 120                 125
   ttc ttt gat cag tgc att gag gaa gaa cca gaa aaa gat ttc cgc ggc      975
   Phe Phe Asp Gln Cys Ile Glu Glu Glu Pro Glu Lys Asp Phe Arg Gly
                   130                 135                 140
   tcg cac ttt cag aat gcg gct agt gag tac cct cgc ccc gaa act gat     1023
   Ser His Phe Gln Asn Ala Ala Ser Glu Tyr Pro Arg Pro Glu Thr Asp
               145                 150                 155
   agc gaa aag ggc att gtt gca gca gtg tta gag cac cgc gag tgg tgt     1071
   Ser Glu Lys Gly Ile Val Ala Ala Val Leu Glu His Arg Glu Trp Cys
           160                 165                 170
   cat aag act ctg act gat ttg ctc act gag aag aac ggc tac cca ggc     1119
   His Lys Thr Leu Thr Asp Leu Leu Thr Glu Lys Asn Gly Tyr Pro Gly
       175                 180                 185
   acc acc cag gcg aat cag ctg ttg gtg ttc ctt gat ggt gga ctt gct     1167
   Thr Thr Gln Ala Asn Gln Leu Leu Val Phe Leu Asp Gly Gly Leu Ala
   190                 195                 200                 205
   gga tct cga ttg gtc cac aac atc agt cct ctt gag acg gct cgc gat     1215
   Gly Ser Arg Leu Val His Asn Ile Ser Pro Leu Glu Thr Ala Arg Asp
                   210                 215                 220
   ttg gct cgg cag ttg ttg tcg gct cca cct gcg gac tac tca att         1260
   Leu Ala Arg Gln Leu Leu Ser Ala Pro Pro Ala Asp Tyr Ser Ile
               225                 230                 235
   tagtttcttc attttccgaa ggggtatctt cgttggggga ggcgtcgata agccccttct   1320
   ttttagcttt aacctcagcg cgacgctgct ttaagcgctg catggcggcg cggttcattt   1380
   cacgttgcgt ttcgcgcctc ttgttcgcga tttctttgcg ggcctgtttt gcttcgttga   1440
   tttcggcagt acgggttttg gtgagttcca cgtttgttgc gtgaagcgtt gaggcgttcc   1500
   atggggtgag aatcatcagg gcgcggtttt tgcgtcgtgt ccacaggaag atgcgctttt   1560
   ctttttgttt tgcgcggtag atgtcgcgct gctctaggtg gtgcactttg aaatcgtcgg   1620
   taagtgggta tttgcgttcc aaaatgacca tcatgatgat tgtttggagg agcgtccaca   1680
   ggttgttgct gacccaatag agtgcgattg ctgtggggaa tggtcctgtg aggccaaggg   1740
   acagtgggaa gatcggcgcg aggatcgaca tcacgatcat gaacttcagc atgccgttag   1800
   agaatccgga tgcgtaatcg ttggtttgga agctgcggta catggacatc              1850
<210> 6
   <211> 236
   <212> PRT
   <213> Corynebacterium glutamicum,
<400> 6
   Met Ser Ile Leu Val Leu Asp Ser Ser Ser Tyr Leu Glu Thr Asp Gln
   1               5                   10                  15
   Pro Glu Arg Thr Trp Pro Asn Val Ala Ala Ser Ala Ser Gly Lys Ser
               20                  25                  30
   Lys Thr Ser Ala Gly Ala Asn Arg Arg Arg Asn Arg Pro Ser Pro Arg
           35                  40                  45
   Gln Arg Leu Leu Asp Ser Ala Thr Asn Leu Phe Thr Thr Glu Gly Ile
       50                  55                  60
   Arg Val Ile Gly Ile Asp Arg Ile Leu Arg Glu Ala Asp Val Ala Lys
   65                  70                  75                  80
   Ala Ser Leu Tyr Ser Leu Phe Gly Ser Lys Asp Ala Leu Val Ile Ala
                   85                  90                  95
   Tyr Leu Glu Asn Leu Asp Gln Leu Trp Arg Glu Ala Trp Arg Glu Arg
               100                 105                 110
   Thr Val Gly Met Lys Asp Pro Glu Asp Lys Ile Ile Ala Phe Phe Asp
           115                 120                 125
   Gln Cys Ile Glu Glu Glu Pro Glu Lys Asp Phe Arg Gly Ser His Phe
       130                 135                 140
   Gln Asn Ala Ala Ser Glu Tyr Pro Arg Pro Glu Thr Asp Ser Glu Lys
   145                 150                 155                 160
   Gly Ile Val Ala Ala Val Leu Glu His Arg Glu Trp Cys His Lys Thr
                   165                 170                 175
   Leu Thr Asp Leu Leu Thr Glu Lys Asn Gly Tyr Pro Gly Thr Thr Gln
               180                 185                 190
   Ala Asn Gln Leu Leu Val Phe Leu Asp Gly Gly Leu Ala Gly Ser Arg
           195                 200                 205
   Leu Val His Asn Ile Ser Pro Leu Glu Thr Ala Arg Asp Leu Ala Arg
       210                 215                 220
   Gln Leu Leu Ser Ala Pro Pro Ala Asp Tyr Ser Ile
   225                 230                 235
<210> 7
   <211> 32
   <212> DNA
   <213> Künstliche Sequenz
<220>
   <221> misc_feature
   <223> Primer metD-DelA
<400> 7
   gatctaaagc ttgcctctcc aatctccact ga 32
<210> 8
   <211> 48
   <212> DNA
   <213> Künstliche Sequenz
<220>
   <221> misc_feature
   <223> Primer metD-DelB
<400> 8
   attgagtagt ccgcaggtgg atttaaataa tccacaggca agtctagc 48
<210> 9
   <211> 48
   <212> DNA
   <213> Künstliche Sequenz
<220>
   <221> misc_feature
   <223> Primer metD-DelC <400> 9

   gctagacttg cctgtggatt atttaaatcc acctgcggac tactcaat 48
<210> 10
   <211> 31
   <212> DNA
   <213> Künstliche Sequenz
<220>
   <221> misc_feature
   <223> Primer metD-DelD <400> 10

   gatctaaagc ttgatgtcca tgtaccgcag c 31
<210> 11
   <211> 1177
   <212> DNA
   <213> Kunstliche Sequenz
<220>
   <221> misc_feature
   <222> (1)..(1177)
   <223> PCR-Produkt: metD-Deletionsderivat
<400> 11
   gatctaaagc ttgcctctcc aatctccact gaggtactta atccttccgg ggaattcggg     60
   cgcttaaatc gagaaattag gccatcacct tttaataaca atacaatgaa taattggaat    120
   aggtcgacac ctttggagcg gagccggtta aaattggcag cattcaccga aagaaaagga    180
   gaaccacatg cttgccctag gttggattac atggatcatt attggtggtc tagctggttg    240
   gattgcctcc aagattaaag gcactgatgc tcagcaagga attttgctga acatagtcgt    300
   cggtattatc ggtggtttgt taggcggctg gctgcttgga atcttcggag tggatgttgc    360
   cggtggcggc ttgatcttca gcttcatcac atgtctgatt ggtgctgtca ttttgctgac    420
   gatcgtgcag ttcttcactc ggaagaagta atctgcttta aatccgtagg gcctgttgat    480
   atttcgatat caacaggcct tttggtcatt ttggggtgga aaaagcgcta gacttgcctg    540
   tggattattt aaatccacct gcggactact caatttagtt tcttcatttt ccgaaggggt    600
   atcttcgttg ggggaggcgt cgataagccc cttcttttta gctttaacct cagcgcgacg    660
   ctgctttaag cgctgcatgg cggcgcggtt catttcacgt tgcgtttcgc gcctcttgtt    720
   cgcgatttct ttgcgggcct gttttgcttc gttgatttcg gcagtacggg ttttggtgag    780
   ttccacgttt gttgcgtgaa gcgttgaggc gttccatggg gtgagaatca tcagggcgcg    840
   gtttttgcgt cgtgtccaca ggaagatgcg cttttctttt tgttttgcgc ggtagatgtc    900
   gcgctgctct aggtggtgca ctttgaaatc gtcggtaagt gggtatttgc gttccaaaat    960
   gaccatcatg atgattgttt ggaggagcgt ccacaggttg ttgctgaccc aatagagtgc   1020
   gattgctgtg gggaatggtc ctgtgaggcc aagggacagt gggaagatcg gcgcgaggat   1080
   cgacatcacg atcatgaact tcagcatgcc gttagagaat ccggatgcgt aatcgttggt   1140
   ttggaagctg cggtacatgg acatcaagct ttagatc                            1177